# EUROPÄISCHE PATENTSCHRIFT

(11) **EP 3 454 820 B1**
(45) Veröffentlichungstag und Bekanntmachung des Hinweises auf die Patenterteilung: **25.08.2021**
(21) Anmeldenummer: 17723037.2
(22) Anmeldetag: 02.05.2017
(51) Int. Cl.: A61K 8/02, A61K 8/06, A61K 8/37, A61K 8/44, A61K 8/60, A61Q 19/10, A61Q 19/00, C11D 1/825, A61K 8/34

(54) **MIKROEMULSIONSKONZENTRATE MIT AMPHOTEREN TENSIDEN**
MICROEMULSION CONCENTRATES WITH AMPHOTERIC SURFACTANTS
CONCENTRES DES MICRO-EMULSIONS COMPRENANT DES SURFACTANTS AMPHOTERES

(30) Priorität: 10.05.2016 EP 16168939
(43) Veröffentlichungstag der Anmeldung: 20.03.2019
(73) Patentinhaber: BASF SE, 67056 Ludwigshafen am Rhein (DE)
(72) Erfinder: HLOUCHA, Matthias, 40589 Düsseldorf-Holthausen (DE); SEIDLER, Stefanie, 40589 Duesseldorf-Holthausen (DE); KUESTERS, Esther, 40589 Düsseldorf-Holthausen (DE); SCHULTE, Petra, 40589 Düsseldorf-Holthausen (DE); STRAUSS, Gabriele, 40589 Duesseldorf-Holthausen (DE)
(74) Vertreter: BASF IP Association
(86) Internationale Anmeldenummer: PCT/EP2017/060323
(87) Internationale Veröffentlichungsnummer: WO 2017/194339

(56) Entgegenhaltungen:
- WO-A2-02/072052
- DE-A1- 10 161 885
- DE-A1- 10 162 184
- DE-A1- 10 205 296
- DE-A1-102011 015 192
- DATABASE GNPD [Online] MINTEL; 1. April 2016 (2016-04-01), EcoWipes: "Soothing Eye Make-Up Removal Pads", XP002759607, Database accession no. 3942023
- DATABASE GNPD [Online] MINTEL; 1. Oktober 2014 (2014-10-01), Bourjois: "Cleansing Wipes", XP002759608, Database accession no. 2756369
- DATABASE GNPD [Online] MINTEL; 1. November 2015 (2015-11-01), Maison Absolution: "L'Eau de Soir et Matin Certified Organic Cleansing Water for Eyes and Face", XP002759609, Database accession no. 3638893

## Beschreibung

### Gebiet der Erfindung

Die Erfindung liegt auf dem Gebiet der kosmetischen Mittel, die in Form von niedrigviskosen Mikroemulsionskonzentraten nach der Verdünnung zur Imprägnierung von Textil und Papier für die Kosmetik geeignet sind sowie die Herstellung derselben.

### Stand der Technik

Flächengebilde auf Basis von Textil und/oder Papier haben als Kosmetiktücher, Baby-Reinigungstücher usw., gemeinhin auch als Wet Wipes bezeichnet, eine breite Anwendung gefunden, die hohen und auch widersprüchlichen Ansprüchen des Verbrauchers genügen müssen. So sollen die auf den Flächengebilden aufgetragenen Mittel zum einen eine gute Reinigungsleistung erbringen, aber zum anderen auch die Haut pflegen und konditionieren, und insbesondere, wenn die Mittel auf der Haut verbleiben, extrem hautfreundlich und frei von hautirritierenden Bestandteilen sein.

Weitere Anforderungen ergeben sich durch die Hersteller solcher Flächengebilde. So wünschen sich die Hersteller Mittel, die möglichst hochkonzentriert sind, um Lager- und Transportkosten zu sparen, und gleichzeitig sollen diese hochkonzentrierten Mittel transparent und niedrigviskos sein und vor allen Dingen während der Verdünnung mit Wasser auf den angewendeten Konzentrationsbereich auch bleiben, ohne dass es zu Vergelungen kommt. Schließlich sollen die wässrigen Verdünnungen selber niedrigviskos und sprühbar sein. Weiterhin erwarten die Hersteller eine hohe Lagerstabilität bei niedrigen und hohen Temperaturen sowohl von den kosmetischen konzentrierten Mitteln als auch von deren Verdünnungen, die zudem unabhängig von Parfum und Konservierungsmittel gegeben sein soll.

Mikroemulsionen sind makroskopisch homogene, optisch transparente Mischungen von zwei nicht miteinander mischbaren Flüssigkeiten. In der Regel handelt es sich dabei um thermodynamisch stabile Emulsionen aus Öl und Wasser, die in Anwesenheit von ausgewählten Emulgatoren in ausgewählten Mengen hergestellt werden.

Die mittleren Teilchengrößen von Mikroemulsionen liegen üblicherweise unter 100 nm und weisen eine hohe Transparenz auf, wodurch sie sich von üblichen Öl-in-Wasser-Emulsionen unterscheiden.

In der Europäischen Patentanmeldung EP 1813251 werden kaltherstellbare mikroemulsionsartige Konzentrate beschrieben, die Mischungen aus einem nichtionischen Emulgator und einem sekundären Emulgator mit ggf. neutralisierter Säurefunktion enthalten, sowie ein Co-Tensid und ein oder mehrere Öle. Die erhaltenen Mikroemulsionskonzentrate sind klar bis transparent und weisen einen Gesamtwassergehalt größer oder gleich 15 bis 60 Gew.-% auf. Weder die Konzentrate noch die wässrigen Verdünnungen sehen den Zusatz weiterer Tenside vor.

Aus der Internationalen Patentanmeldung WO 98/24409 sind schäumende Körperreinigungsmittel bekannt, die neben hautverträglichen anionischen Tensiden (A), Alkylpolyglykoside (B) sowie eine Kombination aus einem zwitterionischen Tensid (C) und einem ampholytischen Tensid (D) zur Verbesserung der Schäumeigenschaften und der Viskosität bestehen.

Die Internationale Patentanmeldung WO 02/072052 (EP1372599) beschreibt Emulsionen, die verdünnt zum Einsatz auf Wipes kommen können. Die Emulgatoren sind ethoxylierte Verbindungen und die Öle sind Mineralöle, die jedoch von Verbrauchern der "grünen" Kosmetik nicht gewünscht werden. In den Verdünnungen der Emulsionen werden Betaine als zwitterionische Tenside zugesetzt.

Die Internationale Patentanmeldung WO 03/066017 offenbart Waschzubereitungen mit Pflanzenölen, Alkylpolyglykosiden und amphoteren Tensiden, die als Ölduschbad angewendet werden. Derartige Ölduschbäder werden bei Zusatz von Wasser milchig-weiß. Es handelt sich nicht um klare, transparente Mikroemulsionen bzw. die wässrigen Verdünnungen führen nicht zu klaren, transparenten Emulsionen.

In der Internationalen Anmeldung WO 2008/155075 werden kosmetische Zubereitungen beschrieben, die neben nicht-alkoxylierten Tensiden ausgewählt aus der Gruppe der anionischen und zwitterionischen oder ampholytischen Tenside eine Mikroemulsion und mindestens ein kationisches Polymer enthalten. Diese Zubereitungen werden eingesetzt als Konditionierungsmittel in Shampoo und Haarbehandlungsmitteln. Zur besseren Konditionierwirkung ist ein kationisches Polymer zwingend. In den Beispielen werden ausschließlich Shampoos offenbart, die neben ethoxylierten anionischen Tensiden Cocoamidopropylbetain und eine Mikroemulsion enthalten. Derartige Shampoos enthalten einen hohen Anteil an schäumenden Tensiden, und kommen daher nicht als Mittel für die Imprägnierung von Tüchern und Papier in der Kosmetikindustrie in Frage. Des Weiteren enthalten die Zubereitungen einen deutlich höheren Anteil an Wasser sowohl in der Mikroemulsion als auch in dem gebrauchsfertigen Haarshampoo, so dass keine Konzentrate vorliegen.

In der Deutschen Patentanmeldung DE 10161885 werden Reinigungsprodukte auf Basis ölhaltiger Mikroemulsionen beschrieben, die neben einem Primärtensid und einem oder mehreren Co-Tensiden und ggf. W/O-Emulgatoren flüssige oder feste Ölphasen enthalten. Die beschriebenen Produkte enthalten alle einen hohen Wassergehalt.

Aus der Deutschen Patentanmeldung DE 10162184 sind Imprägnierlösungen für Kosmetiktücher bekannt, die eine Emulgatormischung aus Alkylpolyglucosiden und Betainen sowie eine Mischung aus Wachskörper, Partialglyceride und Fettalkoholethoxylate neben einem Kationpolymer enthalten. Die Lösungen sind keine Konzentrate und enthalten keine Öle und Amphoacetate.

Die Aufgabe der vorliegenden Erfindung hat darin bestanden, konzentrierte Mittel zur Imprägnierung von Textil und Papier bereit zu stellen, die
- als Konzentrat mit einem Wassergehalt unter 35, vorzugsweise 25 Gew.-% transparent und niedrigviskos sind,
- als Konzentrat ausgezeichnet lagerstabil über einen weiten Temperaturbereich sind,
- nur wenig bis keine ethoxylierten Verbindungen enthalten,
- keine oder nur geringe Mengen an Konservierungsmittel benötigen,
- tolerabel verschiedene Konservierungsmittel und Parfümöle ohne Auswirkung auf die Lagerstabilität vertragen,
- hautfreundliche wenig-schäumende Tenside im Konzentrat enthalten,
- problemlos mit Wasser verdünnbar sind, ohne dass es zu Vergelungen kommt oder zum Brechen der Emulsion,
- bei Raumtemperatur mit Wasser verdünnbar sind, damit der Kunde nicht die Emulsion erwärmen muss,
- nach der Verdünnung mit Wasser zur Imprägnierung der Flächengebilde unter schwankenden Temperaturbelastungen lagerstabil sind,
- nach der Verdünnung mit Wasser sehr niedrigviskos sind, mit Viskositäten möglichst unter 400 oder sogar unter 200 mPas,
- in der Verdünnung Textil und Papier gut benetzen.

Die imprägnierten Flächengebilde aus Textil und Papier sollten bei der Anwendung für den Verbraucher eine milde Reinigungsleistung mit einem angenehmen Hautgefühl erzeugen. Des Weiteren sollten die imprägnierten Flächengebilde auch nach Öffnung über eine lange Zeit die Feuchtigkeit vorhalten, damit der Verbraucher ein feuchtes und nicht ausgetrocknetes Flächengebilde nutzen kann.

Überraschenderweise wurde gefunden, dass konzentrierte Mikroemulsionen mit ausgewählten Emulgatoren in Kombination mit ausgewählten Mengen an Amphoacetaten als speziellem zwitterionischen bzw. amphoteren Tensid die komplexe Aufgabe erfüllen.

Mikroemulsionskonzentrate für die Imprägnierung von Textil und Papier in der Kosmetik enthaltend
a1) Alkyl- und/oder Alkenylglykoside
a2) Co-Tenside ausgewählt aus der von Monoester des Glycerins mit einer C₆-C₂₂-Fettsäure gebildeten Gruppe in Mengen von 3 bis 20 Gew.-% - bezogen auf Konzentrat-
a3) Öle verschieden von a2)
a4) amphoteres bzw. zwitterionisches Tensid ausgewählt aus der Gruppe der Amphoacetate in Mengen von 4,75 bis 6,5 Gew.-% - bezogen auf Konzentrat - und
a5) Wasser,
wobei der Gesamtwassergehalt 0,5 - 35 Gew.-% - bezogen auf Konzentrat - beträgt.

Weitere Gegenstände der Erfindung betreffen die Verwendung der Alkyl- und/oder Alkenylglykoside und Amphoacetate als Emulgator- bzw. Tensidmischung zur Herstellung derartiger Mikroemulsionskonzentrate, ein Verfahren zu der Herstellung derartiger Mikroemulsionskonzentrate, die Verwendung derselben in Form seiner wässrigen Verdünnungen als Imprägniermittel zur Herstellung von Flächengebilden aus Textil und Papier in der Kosmetik, insbesondere von sog. Wet Wipes als auch das Imprägniermittel selber.

Die Mikroemulsionskonzentrate gemäß der vorliegenden Lehre sind vorzugsweise vom Typ Öl-in-Wasser (O/W) oder haben eine bikontinuierliche Struktur. Bevorzugt enthalten die Mikroemulsionskonzentrate als kontinuierliche Phase Wasser (a5), mindestens ein Alkyl- und/oder Alkenyl(oligo)glycosid (a1), ein amphoteres bzw. zwitterionisches Tensid aus der Gruppe der Amphoacetate (a4) und ein davon unterschiedliches Co-Tensid (a2), sowie mindestens ein nicht-wasserlösliches Öl (a3) als innere Phase.

Mikroemulsionskonzentrate sind makroskopisch homogene, optisch transparente, niedrigviskose, thermodynamisch stabile Mischungen.

Die mittleren Teilchengrößen liegen üblicherweise unter 100 nm, vorzugsweise zwischen 3 und 100 nm, bestimmt nach der DLS-Methode mit einem Gerät der Bezeichnung Horiba LB-500. Sie weisen eine hohe Transparenz auf und sind beim Zentrifugieren bei 2000 UPM für mindestens 30 Minuten gegenüber einer sichtbaren Phasenseparation stabil.

Die Leitfähigkeit der erfindungsgemäßen Mikroemulsionskonzentrate liegt vorzugsweise im Bereich von größer/gleich 500 µSi/cm und besonders bevorzugt bei größer/gleich 1000 µSi/cm

Die Transparenz wurde gemessen als FNU Formazine Nephelometric Units - Streulichtmessung (Winkel 90° ) gemäß den Vorschriften der Norm ISO 7027 mit Formazin als Trübungsstandardflüssigkeit. Im Sinne der Erfindung ist eine Zubereitung bis maximal 200 FNU gemessen nach der Norm als transparent zu bezeichnen.

Der Begriff "niedrigviskos" steht in der vorliegenden Anmeldung für Viskositäten gemessen nach Brookfield/RVT/23° C+/-3° C/Sp 1/20rpm unter 500mPas. Vorzugsweise weisen sie Viskositäten im Bereich von 1 bis 400 mPas bestimmt gemäß Brookfield/ RVT/23° C+/-3° C/Sp 1/20rpm auf.

Bei den erfindungsgemäßen sogenannten Ölen a3) handelt es sich um organische, nichtwasserlösliche Ölkomponenten, die bei Raumtemperatur flüssig sind, das bedeutet, die einen Schmelzpunkt unter den üblichen Raumtemperaturen von ca. 18 bis 25 ° C aufweisen.

Im Sinne der vorliegenden Erfindung werden als Öle nichtwasserlösliche Ölkomponenten verstanden, die in Wasser bei 20 ° C eine Löslichkeit von kleiner gleich 2 Gew.-% aufweisen.

Der Begriff "Öle" und "nichtwasserlösliche Ölkomponenten" wird synonym verwendet.

Auch der Begriff der "amphoteren" und "zwitterionischen" Tenside wird synonym verwendet und bezeichnet wie allgemein üblich Tenside, die eine negativ als auch eine positiv geladene funktionelle Gruppe besitzen bzw. in solche durch pH-Wert-Einstellung überführt werden. Die tensidische Natur ergibt sich durch den Strukturaufbau des unpolaren und polaren Teils.

### a1) Alkyl- und/oder Alkenyloligoglykoside

Die Mikroemulsionskonzentrate enthalten zwingend a1) Alkyl- und/oder Alkenyloligoglykoside (im Folgenden auch als "APG" bezeichnet) als Tenside.

Alkyl- und/oder Alkenyloligoglucoside im Sinne der vorliegenden Lehre folgen dabei vorzugsweise der Formel (I),

R¹O-[G]ₚ (I)

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und man hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad größer als 1,1 und kleiner als 1,7 ist und insbesondere zwischen 1,2 und 1,4 liegt.

Einer Ausführungsform entsprechend leitet sich der Alkyl- bzw. Alkenylrest R¹ von primären höheren Alkoholen mit 12 bis 22, vorzugsweise 12 bis 14 Kohlenstoffatomen ab. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol.

Hervorragend ist das Alkyloligoglucosid, welches unter dem Handelsnamen Plantacare® 1200 UP (INCI: Lauryl Glucoside) von der BASF vertrieben wird.

Die Mikroemulsionskonzentrate im Sinne der vorliegenden Lehre enthalten die Komponente (a1) vorzugsweise in Mengen von 5 bis 20 Gew.-%, bevorzugt in Mengen von 7 bis 12 Gew.-% - bezogen auf das Gesamtgewicht der Mikroemulsionskonzentrate.

### a2) Co-Tensid: Monoester des Glycerins

Im Sinne der vorliegenden Erfindung enthalten die erfindungsgemäßen Mikroemulsionskonzentrate zusätzlich als Co-Tensid (a2) Monoester des Glycerins mit einer C₆-C₂₂-Fettsäure.

Bevorzugt handelt es sich dabei um Monoester des Glycerins von C₆-C₂₂-Fettsäuremischungen, die ungesättigte C₁₂-C₂₂-Fettsäuren enthalten. Besonders bevorzugt im Sinne der Erfindung sind Glycerinmonoester von ungesättigten geradkettigen C₁₂-C₂₂-Fettsäuren und insbesondere geeignet ist Glycerinmonooleat. Im Sinne der Erfindung sind sowohl das reine Glycerinmonooleat als auch deren technische Qualitäten geeignet. Ein technisches Produkt ist beispielsweise Monomuls® 90-0 18, ein Handelsprodukt der BASF Personal Care & Nutrition GmbH.

Im Sinne der vorliegenden Erfindung sind die Monoester des Glycerins als Co-Tensid a2) in Mengen von 3 bis 20 Gew.-%, bevorzugt von 3 bis 7 Gew.-% - bezogen auf Mikroemulsionskonzentrat - enthalten.

### a3) Öle

Als zwingender Bestandteil a3) sind Öle enthalten, die vorzugsweise eine Polarität zwischen 5 und 60 mN/m aufweisen. Die Grenzflächenspannung wird bestimmt als Grenzflächenspannung in mN/m in Analogie der ASTM-Methode D971-99a (2004). Die Öle a3) sind verschieden von a2).

Geeignete Verbindungen a3) sind beispielsweise ausgewählt aus der Gruppe, die gebildet wird von
- Guerbetalkoholen auf Basis von Fettalkoholen mit 6 bis 18 C-Atomen,
- Ester linearer C₆-C₂₂-Fettsäuren mit linearen C₆-C₂₂-Fettalkoholen,
- Ester von linearen C₆-C₂₂-Fettalkoholen mit verzweigten C₆-C₃₂-Carbonsäuren,
- Ester von verzweigten C₃-C₁₈ Alkoholen mit linearen C₆-C₃₂-Fettsäuren,
- Ester von verzweigten C₃-C₁₈ Alkoholen mit verzweigten C₆-C₃₂- Carbonsäuren
- Triglyceriden auf Basis C₆-C₁₄-Fettsäuren,
- Ester von C₂-C₁₂-Dicarbonsäuren mit linearen Alkoholen mit 1 bis 22 Kohlenstoffatomen oder Polyolen mit 2 bis 10 Kohlenstoffatomen und 2 bis 6 Hydroxylgruppen,
- pflanzlichen Ölen,
- linearen oder verzweigten C₆-C₂₂-Dialkylcarbonaten,
- linearen oder verzweigten, symmetrischen und/oder unsymmetrischen Dialkylethern mit 6 bis 22 Kohlenstoffatomen pro Alkylgruppe,
- "CX-Kohlenwasserstoffe" der Kohlenwasserstoffgruppe umfassend C7, C9, C11, C13, C15, C17, C19, C21 und/oder C23 Kohlenwasserstoffe.

Beispiele für die Gruppe der Ester von linearen C₆-C₂₂-Fettsäuren mit linearen C₆-C₂₂-Fettalkoholen sind, Caprylylcaprylat, Myristylmyristat, Myristylpalmitat, Myristylstearat, Myristyloleat, Myristylbehenat, Myristylerucat, Cetylmyristat, Cetylpalmitat, Cetylstearat, Cetyloleat, Cetylbehenat, Cetylerucat, Stearylmyristat, Stearylpalmitat, Stearylstearat, Stearyloleat, Stearylbehenat, Stearylerucat, Oleylmyristat, Oleylpalmitat, Oleylstearat, Oleyloleat, Oleylbehenat, Oleylerucat, Behenylmyristat, Behenylpalmitat, Behenylstearat, Behenyloleat, Behenylbehenat, Behenylerucat, Erucylmyristat, Erucylpalmitat, Erucylstearat, Erucyloleat, E-rucylbehenat und Erucylerucat oder deren Mischungen.

Beispiele für die Gruppe der Ester von linearen C₆-C₂₂-Fettalkoholen mit verzweigten C₆-C₃₂-Carbonsäuren sind Myristylisostearat, Cetylisostearat, Cetylisononat, Stearylisononat, Stearylisostearat, Cetylisostearat, Behenylisostearat und Erucylisostearat oder deren Mischungen. Besonders bevorzugt sind hierbei Cetylisononat und/oder Stearylisononat, d.h. Isononansäureester von Cetyl-und/oder Stearylalkohol wie Cetiol® SN, ein Handelsprodukt der BASF Personal Care Nutrition GmbH.

Beispiele für die Gruppe der Ester von verzweigten C₃-C₁₈ Alkoholen mit linearen C₆-C₃₂-Fettsäuren sind Isostearylmyristat, Isostearylpalmitat, Isostearylstearat, Isostearyloleat, Isostearylbehenat, Isopropylpalmitat, Isopropylstearat, Ethylhexylpalmitat, Ethylhexylstearat oder deren Mischungen.

Ein Beispiel für Ester von verzweigten C₃-C₁₈ Alkoholen mit verzweigten C₆-C₃₂- Carbonsäuren ist Isostearylisostearat.

Als Triglyceride eignen sich insbesondere Glycerinester von Fettsäuren mit 8 und/oder 10 Kohlenstoffatome, wobei die Triglyceridester auch Anteile an Diglyceridester enthalten können, beispielsweise Triglycerid-/Diglyceridester wie sie erhältlich sind unter dem Handelsnamen Myritol® 312 von der BASF Personal Care & Nutrition GmbH (INCI Name: Caprylic/Capric Triglyceride).

Dioctylmalat ist beispielsweise ein geeigneter Dicarbonsäureester.
Geeignet sind des Weiteren pflanzliche Öle wie Erdnussöl, Rizinusöl, Kokosnussöl, Maisöl, Olivenöl, Palmkernöl, Sonnenblumenöl, Sojaöl, Rapsöl, Mandelöl, Traubenkernöl, Distelöl, Weizenkeimöl, Nachtkerzenöl, Macadamianussöl, Arganöl und/oder Avocadoöl.

Sehr gute Ergebnisse werden mit linearen oder verzweigten Dialkylcarbonaten als a3) erhalten, vorzugsweise mit symmetrischen linearen oder verzweigten C₆-C₂₂-Dialkylcarbonaten. Beispiele für symmetrische lineare C₆-C₂₂-Dialkylcarbonaten sind Di-n-hexylcarbonat, Di-n-caprylcarbonat, Di-n-hexylcarbonat und/oder deren Mischungen, insbesondere Dicaprylcarbonat wie Cetiol® CC, ein Handelsprodukt der BASF Personal Care & Nutrition GmbH.

Geeignete verzweigte C₆-C₂₂- Dialkylcarbonate können folgende Alkylreste aufweisen: 2-Methylpropyl-, iso-Butyl-, iso-Pentyl, wie z.B. 2,2,-Dimethylpropyl (= Neopentyl), 3-Methylbutyl-(=iso-Pentyl-), iso-Hexyl-, i-Octyl, wie z.B. 2-Ethyl-hexyl- oder 3-Ethylhexyl oder 4-Ethylhexyl- oder 5-Ethylhexyl Reste, i-Decyl-Reste wie z.B. Trimethylheptyl-Rest (= Neodecyl-Rest), Isostearyl-, Isooctyl-, Isononyl-, Isodecyl-, Isotridecyl-, 2-Ethylbutyl-, 2-Ethylhexyl-, 2-Propy-Iheptyl, 2-Butyloctyl, 2-Butyldecyl-, 2-Hexyloctyl-, 2-Hexyldecyl-, 2-Hexyldodecyl-, 2-Octyldecyl-Rest. Insbesondere sind symmetirsche, verzweigte Dialkylcarbonate wie Di-(2- Propyl-1-heptyl)carbonat bzw. Di-(2-Ethyl-1-butyl)carbonat bevorzugt.

Innerhalb der linearen oder verzweigten, symmetrischen und/oder unsymmetrischen Dialkylether werden besonders die symmetrischen Dialkylether bevorzugt, insbesondere Dialkylether mit 6 bis 10 Kohlenstoffatomen pro Alkylgruppe, beispielsweise ein Di-n-Caprylether wie Cetiol OE, ein Handelsprodukt der BASF Personal Care & Nutrition GmbH.

Weiterhin kann a3) aus sogenannten "CX-Kohlenwasserstoffe" bestehen oder diese enthalten, wobei diese "CX-Kohlenwasserstoffe" nur aus Kohlenstoff und Wasserstoff bestehen mit einer C-Zahl von X (wobei X eine ganze Zahl darstellt). So umfasst beispielsweise der Begriff C11-Kohlenwasserstoff alle Kohlenwasserstoffe mit einer C-Zahl von 11. Der Begriff "Kohlenstoff Zahl" oder "C-Zahl" umfasst alle im Kohlenwasserstoff vorhandenen C-Atome. Er beträgt somit z.B. für Undecan = 11 oder für Tridecan = 13.

Gemäß der vorliegenden Erfindung kann a3) aus "CX-Kohlenwasserstoffe" der Kohlenwasserstoffgruppe umfassend C7, C9, C11, C13, C15, C17, C19, C21 und/oder C23 Kohlenwasserstoffe bestehen oder diese enthalten. Vorzugsweise handelt es sich bei den "CX-Kohlenwasserstoffen" um gesättigte und insbesondere um gesättigte und lineare Kohlenwasserstoffe, ausgewählt aus der Gruppe bestehend aus C7, C9, C11, C13, C15, C17, C19, C21 und/oder C23 Kohlenwasserstoffe. Hervorragend geeignete Beispiele sind n-Heptan, n-Nonan, n-Undecan, n-Tridecan, n-Pentadecan, n-Heptadecan, n-Nonadecan, n-Henicosan und/oder n-Tricosan und insbesondere n-Undecan und/oder n-Tridecan.

Bevorzugt im Sinne der Erfindung sind die Öle (a3), die gebildet werden aus der Gruppe der Ester von verzweigten C₆-C₃₂-Carbonsäuren mit linearen C₆-C₂₂ Fettalkoholen, lineare C₆-C₁₂-Dialkylether, symmetrische lineare C₆-C₂₂-Fettalkoholdicarbonate, symmetrische verzweigte C₆-C₂₂-Dialkylcarbonate und/oder gesättigte und lineare "CX" Kohlenwasserstoffe, ausgewählt aus der Gruppe umfassend C7, C9, C11, C13, C15, C17, C19, C21 und/oder C23 Kohlenwasserstoffe.

Ganz besonders bevorzugt sind als Öle (a3) Isononansäureester von Cetyl-und/oder Stearylalkohol wie Cetiol® SN, ein Handelsprodukt der BASF Personal Care Nutrition GmbH, Dicaprylether wie Cetiol® OE, ein Handelsprodukt der BASF Personal Care & Nutrition GmbH, Dicaprylcarbonat wie Cetiol® CC, ein Handelsprodukt der BASF Personal Care & Nutrition GmbH, Di-(2- Propyl-1-heptyl)carbonat wie Cetiol® 4 All, ein Handelsprodukt der BASF Personal Care & Nutrition GmbH sowie n-Undecan und/oder n-Tridecan wie Cetiol® Ulimate, ein Handelsprodukt der BASF Personal Care & Nutrition GmbH.

Insbesondere bevorzugt sind als Öle (a3) solche ausgewählt aus der von Ester von C₆-C₂₂-Fettsäuren mit verzweigten C₆-C₃₂-Carbonsäuren, linearen C₆-C₁₂-Dialkylether und/oder symmetrischen, linearen C₆-C₂₂-Fettalkoholdicarbonate oder deren Mischungen gebildeten Gruppe.

Die Öle können auch feste Fette und/oder Wachse enthalten, sofern insgesamt a3) bei Raumtemperatur als Flüssigkeit vorliegt bzw. bei Raumtemperatur rühr- bzw. verarbeitbar ist. Typische Beispiele für Fette sind Glyceride, d.h. feste oder flüssige pflanzliche oder tierische Produkte, die im Wesentlichen aus gemischten Glycerinestern höherer Fettsäuren bestehen. Hier sind insbesondere feste Mono- und Diglyceride zu nennen. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente. Die Glycerinmonoester a2) werden dabei aber nicht als Bestandteil der Ölphase a3) betrachtet.

Die Mikroemulsionskonzentrate im Sinne der vorliegenden Lehre enthalten die Öle a3) vorzugsweise in Mengen von 15 bis 35 Gew.-%, vorzugsweise in Mengen von 15 bis 25 Gew.-%, bezogen auf Mikroemulsionskonzentrat.

### a4) Amphotere bzw. zwitterionische Tenside

Im Sinne der Erfindung ist es zwingend, dass die amphoteren bzw. zwitterionischen Tenside ausgewählt sind aus der Gruppe der Amphoactetate. Unter dem Begriff der Amphoacetate werden die Carboxylierungsprodukte der Amidoamine verstanden.

Bevorzugte Amphoacetate folgen der Formel **(II),** 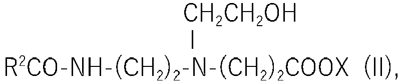 in der R²CO für einen aliphatischen Acylrest mit 6 bis 22 Kohlenstoffatomen und 0 oder 1 bis 3 Doppelbindungen und X für ein Alkalimetall oder Ammonium steht.

Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Eruca-säure sowie deren technische Gemische, mit Aminoethylethanolamin (AEEA) und anschließender Carboxylierung mit Natriumchloracetat. Bevorzugt ist der Einsatz eines Kondensationsproduktes von C_{8/18}-Kokosfettsäure mit Aminoethylethanolamin (AEEA) und anschließender Carboxylierung mit Natriumchloracetat.

Im Sinne der vorliegenden Erfindung sind die Amphoacetate vorzugsweise in den ganz ausgewählten Mengen von 4,75 bis 6,5 Gew.-% - bezogen auf das Konzentrat - enthalten.

### a5) Wasser

Die erfindungsgemäßen Mikroemulsionskonzentrate weisen einen Gesamtwassergehalt von 0,5 bis 35 Gew.-%, vorzugsweise 15 bis 35 und insbesondere 20 bis 25 Gew.-% - bezogen auf Konzentrat - auf.

Dabei zählt für den Gesamtwassergehalt zusätzlich zugegebenes Wasser ebenso wie das Wasser, das als Teil einer wässrigen Formulierung eines aktiven weiteren Bestandteils der Mikroemulsion, beispielsweise von a1), a2) und/oder a3) zugegeben wird.

### a6) Polyole und weitere mögliche Inhaltsstoffe des Mikroemulsionskonzentrats

Die erfindungsgemäßen Mikroemulsionskonzentrate enthalten fakultativ zusätzlich Polyole, vorzugsweise Glycerin, und/oder Konservierungsmittel und/oder Parfum und/oder Salze und/oder pH-Regulantien.

Vorzugsweise sind a6) in Mengen von 0,5 bis 50 und insbesondere in Mengen von 25 bis 40 Gew.-% - bezogen auf Mikroemulsionskonzentrat - enthalten.

Dabei ist es vorteilhaft, wenn Glycerin als Polyol und vorzugsweise in Mengen von 25 bis 38 Gew.-% - bezogen auf Konzentrat - enthalten ist.

Konservierungsmittel sind fakultativ, und vorzugsweise nur in niedrigen Mengen von 0,05 bis 1 Gew.-% enthalten, insbesondere in Mengen von 0,1 bis 0,7 Gew.-%.

Besonders geeignete Konservierungsmittel sind Benzoesäure und deren Salze, Sorbinsäure und deren Salze, Phenoxyethanol, Benzylalkohol, Alkylparabenen, bevorzugt Ethyl-, Methyl- und Propylparaben.

Die pH-Regulantien werden bevorzugt eingesetzt, wenn man einen hautfreundlichen pH-Wert einstellen möchte, der pH-Wert der Mikroemulsionskonzentrate liegt typischerweise zwischen 4,0 bis 5,5 liegt. Zur pH-Wert-Einstellung eignet sich beispielsweise Zitronensäure.

Als Parfüm eignen sich Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohe-xylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfüm, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Ganz besonders bevorzugte Mikroemulsionskonzentrate im Sinne der Erfindung enthalten
a1) 5 bis 20 Gew.-% Alkyl- und/oder Alkenylglykoside
a2) 3 bis 20 Gew.-% Co-Tenside ausgewählt aus der von Monoester des Glycerins mit einer C₆-C₂₂-Fettsäure gebildeten Gruppe
a3) 15 bis 35 Gew.-% Öle verschieden von a2)
a4) 4,75 bis 6,5 Gew.-% amphotere bzw. zwitterionische Tenside ausgewählt aus der Gruppe der Amphoacetate
a5) 15 bis 35 Gew.-% Wasser sowie
a6) 0,5 bis 50 Gew.-% Polyole, vorzugsweise Glycerin und/oder Konservierungsmittel und/oder Parfüm und/oder Salze und/oder pH-Regulantien,
jeweils bezogen auf Konzentrat und in der Summe 100 Gew.-%.

Insbesondere geeignete Mikroemulsionskonzentrate enthalten
a1) 7 bis 12 Gew.-% Alkyl- und/oder Alkenylglykoside
a2) 3 bis 7 Gew.-% Co-Tenside ausgewählt aus der von Monoester des Glycerins mit einer C6-C22-Fettsäure gebildeten Gruppe
a3) 15 bis 25 Gew.-% Öle verschieden von a2)
a4) 4,75 bis 6,5 Gew.-% amphotere bzw. zwitterionische Tenside ausgewählt aus der Gruppe der Amphoacetate
a5) 20 bis 30 Gew.-% Wasser sowie
a6) 25 bis 40 Gew.-% Polyole, vorzugsweise Glycerin, und/oder Konservierungsmittel und/oder Parfüm und/oder Salze und/oder pH-Regulantien,
jeweils bezogen auf Konzentrat ist und in der Summe 100 Gew.-%.

Die erfindungsgemäßen Mikroemulsionskonzentrate können zwar weitere Bestandteile als a1) bis a6) enthalten, aber im Sinne der Erfindung ist es ganz besonders bevorzugt, wenn die erfindungsgemäßen Mikroemulsionen nur aus den beschriebenen Bestandteilen a1) bis a6) in den bevorzugt angegebenen Mengen bestehen. Insbesondere bevorzugt sind Mikroemulsionskonzentrate, die frei sind von Verbindungen, die Ethylenoxid oder andere Alkoxylierungsprodukte enthalten.

### Verfahren zur Herstellung der Mikroemulsionskonzentrate

Ein weiterer Gegenstand der vorliegenden Erfindung ist ein Verfahren zur Herstellung von Mikroemulsionskonzentraten nach Anspruch 1, dadurch gekennzeichnet, dass man eine Mischung herstellt aus a1) Alkyl- und/oder Alkenylglykosiden, a4) amphoteren oder zwitterionischen Tensiden ausgewählt aus der Gruppe der Amphoacetate in Anwesenheit von a5) Wasser und ggf. a6) Polyolen und/oder Konservierungsmittel und/oder Parfum und/oder Salze und/oder pH-Regulantien, diese erwärmt und dazu gleichzeitig oder nacheinander die a3) Öle verschieden von a2) sowie das Co-Tensid a2) ausgewählt aus der von Monoester des Glycerins mit einer C₆-C₂₂-Fettsäure gebildeten Gruppe zugibt.

Einer bevorzugten Ausführungsform entsprechend wird eine Mischung (I) hergestellt aus Alkyl- und/oder Alkenylglykoside a1) und amphoteren bzw. zwitterionischen Tensiden a4) sowie Wasser a5) und Polyole, insbesondere Glycerin als a6). In dieser Mischung (I) können noch die weiteren Inhaltsstoffe aus a6) wie Konservierungsmittel und/oder pH-Regulantien und/oder Parfum und/oder Salze enthalten sein. Das Wasser in der Mischung (I) ergibt sich in der Regel aus den wäßrigen Formulierungen der Alkyl- und/oder Alkenylglykoside a1) und amphoteren bzw. zwitterionischen Tenside a4). Bevorzugt wird die Mischung (I) dann auf etwa 50 bis 80° C, insbesondere 65 bis 75° C unter Rühren erwärmt. Sobald die Mischung (I) klar gelöst vorliegt, wird vorzugsweise dazu nacheinander das Öl a3) und dann das Co-Tensid a2) zugegeben und bevorzugt bei etwa 70 bis 80° C nachgerührt. Es empfiehlt sich eine Nachrührzeit von etwa 10 bis 30 Minuten. Nach Abkühlung kann der pH-Wert eingestellt werden.

Es werden klare, lagerstabile Mikroemulsionskonzentrate erhalten. Dies ist umso überraschender als dies mit Betainen nicht gelingt.

Ein weiterer Gegenstand der vorliegenden Erfindung ist daher die Verwendung einer Mischung aus a1) Alkyl- und/oder Alkenylglykosiden und a4) amphoteren und/oder zwitterionischen Tensiden ausgewählt aus der Gruppe der Amphoacetate als Emulgator- bzw. Tensidmischung zur Herstellung stabiler Mikroemulsionskonzentrate nach Anspruch 1 mit einem Gesamtwassergehalt von 0,5 bis 35 Gew.-%.

Im Sinne der Erfindung hat es sich als vorteilhaft herausgestellt, wenn das Mengenverhältnis von a1): a4) im Bereich von 1,9: 1 bis 1,45: 1 - im Konzentrat - liegt.

Im Sinne der Erfindung ist gerade die Verwendung der Kombination der von a1) und a4) vorteilhaft für die sehr gute Lagerstabilität der erfindungsgemäßen Mikroemulsionskonzentrate.

Die erfindungsgemäßen Mikroemulsionskonzentrate können mit Wasser in beliebige wässrige Verdünnungen überführt werden, ohne dass während der Verdünnung eine störende Vergelung oder ein Brechen der Emulsion erfolgt.

### Mikroemulsionskonzentrate und deren Anwendung

Da die wässrigen Verdünnungen der Mikroemulsionskonzentrate eine überragende Benetzbarkeit von Papier und Textil aufweisen, betrifft ein weiterer Gegenstand der vorliegenden Erfindung die Verwendung der erfindungsgemäßen Mikroemulsionskonzentrate nach einem der Ansprüche 1 bis 10 in Form seiner wässrigen Verdünnung als Imprägniermittel von Papier und Textil zur Herstellung von Flächengebilden in der Kosmetik, insbesondere sog. Wet Wipes.

Bevorzugt sind die erfindungsgemäßen Mikroemulsionskonzentrate in Mengen von 0,1 bis 10 Gew.-% - bezogen auf wässrige Verdünnung bzw. Imprägniermittel - enthalten.

Unter dem Oberbegriff "Textil und Papier" werden verschiedene Sorten und Artikel verstanden, die sich in ihren Anwendungsgebieten und ihrer Beschaffenheit zum Teil erheblich unterscheiden können. Geeignet sind beispielsweise Tissuepapiere und/oder Tissuegewebe und/oder Tissuetücher (im Weiteren mit Tissuetücher bezeichnet). Diese können ein- oder mehrlagig aufgebaut sein. In der Regel weisen die Papiere ein Quadratmetergewicht von 10 bis 65, vorzugsweise 15 bis 30 g und eine Dichte von 0,6 g/cm und weniger auf. Beispiele für Tissuepapiere sind Toilettenpapiere, Papiertaschentücher, Gesichtsreinigungstücher, Abschminktücher, Erfrischungstücher, Haushaltstücher und dergleichen. Neben den papierbasierten Tissues kommen auch entsprechende Tissuegewebe in Frage, die aus Faser- oder Fleecestoff hergestellt werden.

Bevorzugt sind mehrlagige Tissuetücher. Insbesondere sind solche Tissuetücher bevorzugt, welche zwischen den einzelnen Lagen eine undurchlässige und/oder teildurchlässige Sperrschicht haben. Die teildurchlässige Sperrschicht kann beispielsweise als semipermeable Membran ausgebildet sein. Mit derartigen Tüchern können zwei oder mehrere Zusammensetzungen (gegebenenfalls nach vorheriger Verdünnung) auf ein Tuch aufgebracht werden. Dies kann ganz besonders bevorzugt sein, um mit der einen Seite der Tücher die Reinigung mittels der auf das Tuch aufgebrachten Zusammensetzung zu bewirken. Mit der anderen Seite kann dann beispielsweise zum Trocknen nachgerieben werden oder gegebenenfalls ein pflegender Wirkstoff auf die Haut aufgetragen werden.

Weiterhin können die Tücher aus mindestens 3 Lagen mit Zusammensetzungen (gegebenenfalls nach vorheriger Verdünnung) behandelten Tissuetuches bestehen. Vorteilhaft ist dann zwischen mindestens 2 Lagen behandeltem Tuch jeweils 1 Lage Tuch als semipermeable Membran ausgebildet. Die semipermeable Membran ist dabei in Richtung der äußeren Tuchlagen durchlässig. Dadurch kann im Inneren beispielsweise eine Zusammensetzung (gegebenenfalls nach vorheriger Verdünnung) auf die innerste Schicht aufgebracht werden, welche entweder nicht mischbar und/oder nicht stabil mit der auf die äußere Seite aufgetragenen Zusammensetzung ist. Hierdurch wird es möglich "two in one Tücher" zur Reinigung und Pflege anzubieten. Die unterschiedliche farbliche Gestaltung der Tuchlagen sowie der unterschiedliche Aufbau der Tücher aus mehreren Materialien, insbesondere in Bezug auf die Saugfähigkeit und Durchlässigkeit der unterschiedlichen Tuchlagen, ist ebenso möglich.

Weiterhin eignen sich beispielsweise Textilfasern sowohl aus Naturfasern wie z.B. Cellulose, Seide, Wolle, Regeneratcellulose (Viskose, Rayon), Cellulosederivate als auch Textilfasern aus synthetischen Fasern wie z.B. Polyester-, Polypropylen-, Polyethylenterephtalat-, Polyamid-, Polyolefin-, Polyacrylnitril-Fasern oder Mischungen solcher Fasern. Diese Fasern können gewebt oder ungewebt sein.

Der Begriff "Imprägniermittel" umfasst dabei ein Mittel zur Imprägnierung, wobei unter "Imprägnierung" jede Art der Aufbringung einer Flüssigkeit auf mehr oder weniger feste Oberflächen der oben beschriebenen Art gemeint ist, beispielsweise Tränken, Beschichten, Be- oder Ansprühen, Tauchen, Ausrüsten, Abstreifen etc.

Vorzugsweise werden die erfindungsgemäßen Mikroemulsionskonzentrate in Form einer wässrigen Verdünnung als Imprägniermittel verwendet, wobei je nach dem gewünschten Anwendungszweck weitere, zusätzliche kosmetische Wirk- und/oder Hilfsstoffe enthalten sein können. Dabei kann es sich um die gleichen kosmetischen Wirk- und/oder Hilfsstoffe handeln, die bereits unter a6) im Zusammenhang mit den erfindungsgemäßen Mikroemulsionskonzentraten beschrieben worden sind oder auch um weitere übliche, dem Fachmann bekannte Wirk- und Hilfsstoffe, die in wässrigen Verdünnungen auf Textil und Papier aufgebracht werden und als Flächengebilde in der Kosmetik, insbesondere sog. Wet Wipes in den Handel kommen.

Tenside sind typischerweise in Formulierungen für Wet Wipes zur Reinigung der Haut enthalten.

Ein weiterer Gegenstand der vorliegenden Erfindung sind daher Imprägniermittel von Papier und Textil zur Herstellung von Flächengebilden in der Kosmetik enthaltend
A) 0.1 bis 10 Gew.% Mikroemulsionskonzentrate nach Anspruch 1 und
B) Wasser sowie ggf.
C) weitere kosmetische Inhaltsstoffe ausgewählt aus der Gruppe der anionischen/nicht-ionischen, kationischen und/oder zwitterionischen Tenside sowie ggf.
D) weitere Zusatzstoffe verschieden von C).

Vorzugsweise sind die anionischen Tenside ausgewählt aus der Gruppe, die gebildet wird von Seifen, Alkylbenzolsulfonate, Alkansulfonate, Olefinsulfonate, α-Methylestersulfonate, Sulfofettsäuren, Alkylsulfate, Alkylethersulfate, Hydroxymischethersulate, Monoglyceridsulfate, Fettsäureamidsulfate, Mono- und Dialkylsulfosuccinate, Mono- und Dialkylsulfosuccinamate, Sulfotriglyceride, Amidseifen, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkyloligoglucosidcarboxylate, Proteinfettsäurekondensate (insbesondere pflanzliche Produkte auf Weizenbasis) und Alkylphosphate; besonders bevorzugt sind Alkylsulfate alleine oder in Mischung mit weiteren milden anionischen Tensiden wie Acylglutamate und/oder Alkyloligoglucosidcarboxylate.

Alkylsulfate ("Fettalkoholsulfate") stellen bekannte anionische Tenside dar, die großtechnisch durch SO₃- oder Chlorsulfonsäure (CSA)-Sulfatierung von Fettalkohol- und nachfolgende Neutralisation hergestellt werden. Im Sinne der Erfindung kommen Sulfate in Betracht, die der Formel

R⁵O-SO₃X '

folgen, in der R⁵ für einen linearen oder verzweigten Alkyl- und/oder Alkenylrest mit 6 bis 22 Kohlenstoffatomen und X ' für ein Alkali- und/oder Erdalkalimetall, Ammonium, Alkylammonium, Alkanolammonium oder Glucammonium steht. Typische Beispiele sind die Sulfate Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze.

Besonders bevorzugt ist das Natriumsalz des Sulfats auf Basis des Alkohols abgeleitet von der Kokosfettsäure.

Bevorzugt sind von nichtionischen Tensiden Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Bevorzugt sind nichtionische Tenside ohne Ethylenoxid- und/oder Propylenoxid-Einheiten.

Als zwitterionische Tenside werden solche oberflächenaktiven Verbindungen bezeichnet, die im Molekül mindestens eine quartäre Ammoniumgruppe und mindestens eine -COO(-)- oder -S03(-)-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Ampho-acetate und/oder Proteinfettsäurekondensate, letztere vorzugsweise auf Basis von Weizenproteinen bzw. deren Salzen.

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Im Sinne der Erfindung können Tenside mit Alkylenoxid-Einheiten enthalten sein, aber bevorzugt sind ausschließlich Tenside ohne Alkylenoxid-Einheiten enthalten.

Die erfindungsgemäßen Imprägniermittel können falls nach Anwendung gewünscht auch weitere Zusatzstoffe D) enthalten, die dem Fachmann bekannt sind, wie beispielsweise Emulgatoren, Perlglanzwachse, Stabilisatoren, Salz, Verdickungsmittel, Konsistenzgeber, Selbstbräuner, Pigmente, Antioxidationsmittel, Antischuppenmittel, Filmbildner, Quellmittel, Insektenrepellentien, Deodorant- und Antitranspirantwirkstoffe, biogene Wirkstoffe, Feuchthaltemittel (z.B. Glycerin, Sorbitol) und/oder weitere pH-Regulantien. Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaluronsäure, Creatin, Proteinhydrolysate, Pflanzenextrakte, Peptide und Vitaminkomplexe bevorzugt. Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate und hydrophob modifizierte Polyacrylate, Polyacrylamide, Polymere, Polyvinylalkohol und Polyvinylpyrrolidon. Als besonders wirkungsvoll haben sich auch Bentonite erwiesen, bei dem es sich um eine Mischung aus Cyclopentasiloxan, Disteardimonium Hectorit und Propylencarbonat handelt. Weiter in Frage kommen Ester von Fettsäuren mit Polyolen wie beispielsweise Pentaerythrit oder Trimethylolpropan wie Arlypon® TT, ein Handelsprodukt der BASF Personal Care and Nutrition GmbH, sowie Elektrolyte wie Kochsalz und Ammoniumchlorid. Besonders bevorzugt werden als Verdicker Mischungen des Xanthan-Gums.

Falls erneut Konservierungsmittel eingesetzt werden, sind diese bevorzugt ausgewählt aus der schon im Zusammenhang mit den Mikroemulsionskonzentraten genannten Gruppe, beispielsweise Benzoesäure und deren Salze, Zitronensäure und deren Salze, Phenoxyethanol, Benzylalkohol, Alkylparabenen, bevorzugt Ethyl-, Methyl- und Propylparaben oder Formaldehydlösung, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Falls stärkere Parfumierung gewünscht wird, können natürlich weitere Parfüme in den Imprägniermittel enthalten sein, beispielsweise die bereits unter a6) aufgezählten Parfume der Mikroemulsionskonzentrate.

Des Weiteren sind häufig auch wasserlösliche Verdickungsmittel wie natürliche und/oder synthetische Polymere wie Xanthan-Gum, Hydroxyethylcellulose, Polyvinylpyrrolidon oder hochmolekulare Polyethylenoxide als Hilfsstoffe enthalten.

Auch Hydrotrope können zur Verbesserung des Fließverhaltens enthalten sein. Typische Beispiele sind Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin und/oder Dialkoholamine, wie Diethanolamin oder 2-Amino-1,3-propandiol. Falls Glycerin als Hydrotrop gewünscht wird, ist dieses zusätzlich zu der angegebenen Menge Polyol im erfindungsgemäßen Konzentrat zuzugeben.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butyl-aminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil. 108, 95 (1993**)** aufgeführt.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als Insekten-Repellentien kommen N,N-Diethyl-m-toluamid, 1,2-Pentandiol oder Ethyl Butylacetylaminopropionate in Frage.

Als **UV-Lichtschutzfilter** sind bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
3-(4'-Trimethylammonium) benzyliden- bornan-2-on-methylsulfat (Mexoryl SO)
3,3'-(1,4-Phenylendimethin)-bis (7,7- dimethyl-2-oxobicyclo-[2.2.1] heptan-1-methansulfonsäure) and salts (Mexoryl SX)
3-(4'-Sulfo)-benzyliden-bornan-2-on and salts (Mexoryl SL)
Polymer von N-[2(und 4)-(2-oxoborn-3-ylidenmethyl)benzyl]acrylamid Mexoryl SW)
2-(2H-Benzotriazol-2-yl)-4-methyl-6-(2-methyl-3-(1,3,3,3-tetramethyl-1- (trimethylsilyloxy)disiloxanyl)propyl) phenol (Mexoryl XL)
4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
Ester der Salicylsäure, vorzugsweise Salicylsäure-2-ethylhexylester, Salicylsäure-4-isopropylbenzylester, Salicylsäurehomomenthylester;
Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
Ester der Benzalmalonsäure, vorzugsweise 4-Methoxybenzmalonsäuredi-2-ethylhexyl-ester;
Triazinderivate, wie z.B. 2,4,6-Trianilino-(p-carbo-2'-ethyl-l'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenylamino]-1,3,5-triazin-2,4-diyl)diimino] bis(benzoesäure-2- ethylhexylester) (Uvasorb® HEB);
2,2(-Methylen-bis(6-(2H-benzotriazol-2-yl)-4- (1,1,3,3-tetramethyl-butyl)phenol) (Tinosorb M);
2,4-Bis[4-(2-ethylhexyloxy)-2- hydroxyphenyl]-6-(4- methoxyphenyl)-1,3,5- triazin (Tinosorb S);
Propan-1,3-dione, wie z.B. 1-(4-tert.Butylphenyl)-3-(4'methoxyphenyl)propan-1,3-dion;
Ketotricyclo(5.2.1.0)decan-Derivate, wie in der EP 0694521 B1 beschrieben;
Dimethicodiethylbenzalmalonate (Parsol SLX).
Als wasserlösliche UV-Filter kommen in Frage:
2-Phenylbenzimidazol-5-sulfonsäure und deren Alkali-, Erdalkali-, Ammonium-, Alkylammonium-, Alkanolammonium- und Glucammoniumsalze;
2,2(-(1,4-Phenylen)bis(1H-benzimidazol-4,6-disulfonsäure, Mononatriumsalz) (Neo Heliopan AP)
Sulfonsäurederivate von Benzophenonen, vorzugsweise 2-Hydroxy-4-methoxybenzophenon-5-sulfonsäure und ihre Salze;
Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-metho-xydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimt-säure-2-ethylhexylester und/oder 4-Methoxyzimtsäurepropylester und/oder 4-Methoxyzimtsäure-isoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert. Als UV-Lichtschutzfilter eignen sich insbesondere die gemäß Annex VII der Kommissions Direktive (in der Fassung **Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für Zinkoxide sind z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in SÖFW-Journal 122, 8/1996, S. 543-548 sowie Parf. Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. alpha-Carotin, beta-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnS04) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Typische Beispiele für Solubilisatoren sind INCI: Polysorbate 20 (Eumulgin® SML 20), INCI: Polysorbate 80 (Eumulgin® SMO 20), INCI: Polysorbate 60 (Eumulgin® SMS 20), hydriertes Ricinusöl mit verschiedenen Ethoxylierungsgraden, beispielsweise mit 40 EO (Eumulgin® CO 40).

Die erfindungsgemäßen Imprägniermittel enthalten vorzugsweise
A) 0,1 bis 10 Gew.-% Mikroemulsionskonzentrate und
B) 90 bis 99, 8 Gew.-% Wasser
C) 0,05 bis 10 Gew.-% C) und
D) 0,05 bis 10 Gew.-% D).

Die erfindungsgemäßen Imprägniermittel sind in weiten pH-Bereichen lagerstabil und werden vorzugsweise auf pH-Werte von 4,0 bis 7,5 eingestellt.

Die erfindungsgemäßen Imprägniermittel sind optisch homogen, das heißt mit dem Auge werden sie als eine Phase wahrgenommen. Des Weiteren zeigen sie gute Lagerstabilitäten und eine niedrige Viskosität, auch bei langer Lagerung; vorzugsweise liegen die Werte unter 400, insbesondere unter 200mPas.

Die derart imprägnierten Flächengebilde aus Textilien oder Papier werden in der Kosmetik vorzugsweise im Bereich der sog. Wet Wipes zur Hautpflege oder -reinigung (insbesondere der Babypflege oder -reinigung) angewendet. Beispielsweise seien genannt Pflege- oder Reinigungstücher für die Gesichtshaut (sog. Facial tissues, Abschminktücher/Make-up-Entfernung etc.), Erfrischungstücher für die Haut, antibakterielle und/oder desodorierende Tücher, Produkte für die Intimpflege; wie beispielsweise Tampons, Damenbinden, Slipeinlagen, Intimpflegetücher), feuchtes Toilettenpapier, Inkontinenzprodukte, Selbstbräunungstücher oder sog. Insect Repellent Tücher.

### Beispiele

Die Herstellung der Mikroemulsionskonzentrate erfolgte wie folgt:
In einen auf 45° C aufgeheizten Behälter wurden Glycerin, Plantacare® 1200UP, Benzoesäure, ca. 50-60% der Gesamtmenge an Zitronensäurelösung und Dehyton® MC gegeben und mit einer mittleren Rührdrehzahl gerührt, dann wurde auf 70° C erhitzt. Als alles klar gelöst vorlag, wurde Cetiol® SN und Monomuls® 90-018 zugegeben und bei 75 ° C 15 Minuten bis 30 Minuten gerührt. Man ließ auf 50° C abkühlen und gab die restliche Menge an Zitronensäurelösung zu.

Weiteres Abkühlen auf ca. 30° C und den pH-Wert prüfen; ggf. nachdosieren mit Zitronensäure-Lösung auf gewünschten pH-Wert, ca. pH 4,5 einstellen.

Eingesetzte Verbindungen:
Plantacare® 1200UP (INCI: Laurylglucosid); Alkypolyglykosid a1)
Cetiol® SN (INCI: Cetearyl Isononanoate); Öl a3)
Dehyton® MC (INCI: Sodium Cocoamphoacetate); amphoteres bzw. Zwitterionisches Tensid a4)
Monomuls® 90-018 (INCI: Glyceryl Oleate) Co-Tensid a2)
Zitronensäurelösung (50 gew.-%ig).
Die Verbindungen wurden in folgenden Mengen eingesetzt:

### A) Mikroemulsionskonzentrate Beispiel 1a- 1e mit verschiedenen Ölen

| **Product** | **INCI** | | **wt.-% (active matter)** | **wt.-% (active matter)** | **wt.-% (active matter)** | **wt.-% (active matter)** | **wt.-% (active matter)** |
|---|---|---|---|---|---|---|---|
| | | | 1a | 1b | 1c | 1d | 1e |
| Cetiol® SN | Cetearyl Isononanoate | 17,00 | 17,00 | | | | |
| Cetiol® CC | Di-n-Octylcarbonate | | | 21 | | | |
| Cetiol® OE | Di-n-Octylether | | | | 21 | | |
| Cetiol® 4 All | Dipropylheptylcarbonate | | | | | 17 | |
| Cetiol® Ultimate | Undecane/Tridecane | | | | | | 25 |
| Monomuls® 90-018 | Glyceryl Oleate | | 4,20 | 3,70 | 3,70 | 4,20 | 3,00 |
| Dehyton® MC | Sodium Cocoamphoacetate | | 5,87 | 5,87 | 5,87 | 5,87 | 5,87 |
| Plantacare® 1200 UP | Lauryl Glucoside | | 9,45 | 9,45 | 9,45 | 9,45 | 9,45 |
| Glycerin | Glycerin | | 37,92 | 34,42 | 34,42 | 37,92 | 31,12 |
| Benzoic Acid | Benzoic Acid | | 0,50 | 0,50 | 0,50 | 0,50 | 0,50 |
| Citric Acid Sol. (50%) | Citric Acid | | 1,84 | 1,84 | 1,84 | 1,84 | 1,84 |
| Sodium Chloride | Sodium Chloride | | 1,25 | 1,25 | 1,25 | 1,25 | 1,25 |
| Water demin. | | | 21,97 | 21,97 | 21,97 | 21,97 | 21,97 |
| sum | | | 100,00 | 100 | 100 | 100 | 100 |

Es entstanden Konzentrate mit ausgezeichneter Lagerstabilität. Nach 4 Wochen Lagerung bei -5° C, 5° C, Raumtemperatur und 40° C waren die Konzentrate noch klar und die Viskosität unverändert. Bei allen Konzentraten war die Transparenz vor und nach der Lagerung gemessen als FNU unter 10. Auch die Viskosität war nach Lagerung < 350 m Pas (Messbedingungen: 23° C, Spindel 1, 20 rpm).
Beispiel 1a) wurde 12 Wochen bei den verschiedenen Temperaturen gelagert; das Konzentrat 1a) war auch nach der 12-wöchiger Lagerung klar und die Viskosität war unverändert.

Mikroemulsionskonzentrate mit Amphoacetat in verschiedenen Gewichts-% (active matter): Erfindungsgemäße Beispiele 2-4 und Vergleichsbeispiele 1+2

| | | Vergleichsbeispiel 1 | Beispiel 2 | Beispiel 3 | Beispiel 4 | Vergleichsbeispiel 2 |
|---|---|---|---|---|---|---|
| | INCI | [%] | [%] | [%] | [%] | [%] |
| Cetiol® SN | Cetearyl Isononanoate | 21,00 | 21,00 | 21,00 | 21,00 | 21,00 |
| Plantacare® 1200UP | Lauryl Glucoside | 9,45 | 9,45 | 9,45 | 9,45 | 9,45 |
| Dehyton® MC | Sodium Cocoamphoacetate | 4,70 | 4,99 | 5,87 | 6,46 | 6,76 |
| Monomuls® 90-018 | Glyceryl Oleate | 5,10 | 5,10 | 5,10 | 5,10 | 5,10 |
| Glycerin | Glycerine | 36,58 | 35,69 | 33,02 | 31,24 | 30,35 |
| Benzoesäure | Benzoic Acid | 0,50 | 0,50 | 0,50 | 0,50 | 0,50 |
| Water dem. | Aqua | 19,83 | 20,37 | 21,97 | 23,04 | 23,57 |
| Sodium Chloride | | 1,00 | 1,06 | 1,25 | 1,37 | 1,43 |
| Zitronensäure-Lsg. | Citric Acid | 1,84 | 1,84 | 1,84 | 1,84 | 1,84 |
| Aussehen des Konzentrats | | trüb; zweiphasig | klar; Mikroemulsion | klar; Mikroemulsion | klar; Mikroemulsion | trüb; zweiphasig |

Die Mikroemulsionen nach den erfindungsgemäßen Beispielen 2, 3 und 4 waren klar und die Viskosität der Mikroemulsionen war auch nach 4 Wochen Lagerung unverändert: < 350 m Pas; (Messbedingungen: 23° C, Spindel 1, 20 rpm).

Mikroemulsion mit anderem Tensid: Vergleichsbeispiel 3 mit Betain als zwitterionischem Tensid

| | INCI | [%] |
|---|---|---|
| Cetiol® SN | Cetearyl Isononanoate | 21,00 |
| Plantacare® 1200UP | Lauryl Glucoside | 9,45 |
| Dehyton® PK45 | Cocamidopropyl Betaine | 5,87 |
| Monomuls® 90-018 | Glyceryl Oleate | 5,10 |
| Glycerin | Glycerine | 34,94 |
| Benzoesäure | Benzoic Acid | 0,50 |
| Water dem. | Aqua | 21,29 |
| Zitronensäure-Lsg. | Citric Acid | 1,84 |
| Aussehen des Konzentrats | | aufgerahmte Emulsion |

| | | |
|---|---|---|
| Gewichts-% (active matter) | | |

### B) Anwendungsbeispiele: Imprägniermittel

Es wurden Imprägniermittel hergestellt enthaltend die Mikroemulsion nach Beispiel 1a). Die % sind als Gewichtsprozent "raw material" angegeben.
Beispiel 5
   5,0% Mikroemulsion nach Beispiel 1a)
   0,5% Sodium Benzoat
   0,1% Parfum Cotton Touch der Fa. Symrise
   0,1% Eumulgin® CO 40 als Solubilisator (= hydriertes Ricinusöl + 40 EO)
   q.s. Citric Acid (50%ig) zur pH-Einstellung pH 4,8-5,1
   ad 100% Wasser, demin.
Beispiel 6
   5,0% Mikroemulsion nach Beispiel 1a)
   0,6% Rheocare® HSP 1180 (Polyacrylamidomethylpropane Sulfonic Acid)
   0,1% Parfum Cotton Touch der Fa. Symrise
   1,0% Phenoxyethanol
   q.s. KOH (20%ig) zur pH-Einstellung pH 6,0-6,5
   ad 100% Wasser, demin.
Beispiel 7
   5,0% Mikroemulsion nach Beispiel 1a)
   0,5% Sodium Benzoat
   0,1% Parfum Cotton Touch der Fa. Symrise
   0,1% Xanthan Gum
   q.s. Citric Acid (50%ig) zur pH-Einstellung pH 4,8-5,1
   ad 100% Wasser, demin.
Beispiel 8
   3,3% Mikroemulsion nach Beispiel 1a)
   0,1% Parfum Cotton Touch der Fa. Symrise
   0,5% Sodium Benzoat
   q.s. Citric Acid (50%ig) zur pH-Einstellung pH 4,8 -5,1
   ad 100% Wasser, demin.
Beispiel 9
   3,3% Mikroemulsion nach Beispiel 1a)
   0,1% Parfum Cotton Touch der Fa. Symrise
   1,0% Phenoxyethanol
   q.s. NaOH-Lsg. (10%ig) zur pH-Einstellung pH 6,0 -7,0
   ad 100% Wasser, demin.
Beispiel 10
   3,3% Mikroemulsion nach Beispiel 1a)
   0,1% Parfum Cotton Touch der Fa. Symrise
   1,0% Euxyl® K700 der Fa. Schülke (Phenoxyethanol, Benzyl Alcohol, Potassium Sorbate, Tocopherol)
   q.s. Citric Acid (50%ig) zur pH-Einstellung pH 4,8 -5,1
   ad 100% Wasser, demin.
Beispiel 11
   5,0% Mikroemulsion nach Beispiel 1a)
   0,5% Sodium Benzoat
   0,1% Parfum Cotton Touch der Fa. Symrise
   0,4% D-Panthenol 75W
   q.s. Citric Acid (50%ig) zur pH-Einstellung pH 4,8-5,1
   ad 100% Wasser, demin.
Beispiel 12
   5,0% Mikroemulsion nach Beispiel 1a)
   0,5% Sodium Benzoat
   0,1% Parfum Cotton Touch der Fa. Symrise
   0,3% Aloe Vera Gel Concenrtrate 10/1 der Fa. Symrise
   q.s. Citric Acid (50%ig) zur pH-Einstellung pH 4,8-5,1
   ad 100% Wasser, demin.

Gemäß den Beispielen 5 bis 12 entstanden jeweils optisch homogene, klare bis trübe Imprägniermittel, die auch nach 4 Wochen Lagerung bei Raumtemperatur erhalten blieb. Des Weiteren blieb auch die Viskosität nach 4 Wochen Lagerung erfreulich niedrig (max. 100mPas gemessen bei RT), so dass auch die Imprägniermittel über eine gute Lagerstabilität verfügen).

## Patentansprüche

1. Mikroemulsionskonzentrate für die Imprägnierung von Textil und Papier in der Kosmetik enthaltend
a1) Alkyl- und/oder Alkenylglykoside
a2) Co-Tenside ausgewählt aus der von Monoester des Glycerins mit einer C₆-C₂₂-Fettsäure gebildeten Gruppe in Mengen von 3 bis 20 Gew.-% - bezogen auf Konzentrat-
a3) Öle verschieden von a2)
a4) amphoteres bzw. zwitterionisches Tensid ausgewählt aus der Gruppe der Amphoacetate in Mengen von 4,75 bis 6,5 Gew.-% - bezogen auf Konzentrat - und
a5) Wasser,
wobei der Gesamtwassergehalt 0,5 - 35 Gew.-% - bezogen auf Konzentrat - beträgt.

2. Mikroemulsionskonzentrate nach Anspruch 1, **dadurch gekennzeichnet, dass** sie
a1) Alkyl- und/oder Alkenylglykoside der Formel (I)
**R**¹**O-[G]ₚ** **(I)**
enthalten, in der R¹ für einen Alkyl- und/oder Alkenylrest mit 4 bis 22 Kohlenstoffatomen,
G für einen Zuckerrest mit 5 oder 6 Kohlenstoffatomen und p für Zahlen von 1 bis 10 steht.

3. Mikroemulsionskonzentrate nach Anspruch 1, **dadurch gekennzeichnet, dass** sie a2) Monoester des Glycerins enthalten, ausgewählt aus der Gruppe, die gebildet wird von Monoester mit einer ungesättigte C₁₂-C₂₂-Fettsäuren enthaltenden C₆-C₂₂-Fett-säuremischung.

4. Mikroemulsionskonzentrate nach Anspruch 1, **dadurch gekennzeichnet, dass** sie a3) Öle enthalten, die gebildet wird aus der Gruppe der Ester von verzweigten C₆-C₃₂-Carbonsäuren mit linearen Fettalkoholen C₆-C₂₂ , lineare C₆-C₁₂-Dialkylether, symmetrische lineare C₆-C₂₂-Fettalkoholdicarbonate, symmetrische verzweigte C₆-C₂₂-Dialkylcarbonate und/oder gesättigte und lineare "CX" Kohlenwasserstoffe ausgewählt aus der Gruppe umfassend C7, C9, C11, C13, C15, C17, C19, C21 und/oder C23 Kohlenwasserstoffe.

5. Mikroemulsionskonzentrate nach Anspruch 1, **dadurch gekennzeichnet, dass** sie a3) Öle enthalten ausgewählt aus der von Ester von C₆-C₂₂-Fettsäuren mit verzweigten C₆-C₃₂-Carbonsäuren, linearen C₆-C₁₂-Dialkylether und/oder linearen symmetrischen C₆-C₂₂-Fettalkoholdicarbonate oder deren Mischungen gebildeten Gruppe.

6. Mikroemulsionskonzentrate nach Anspruch 1, **dadurch gekennzeichnet, dass** sie als a4) Amphoacetate solche der Formel (II), 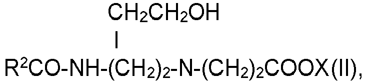 in der R2CO für einen aliphatischen Acylrest mit 6 bis 22 Kohlenstoffatomen und 0 oder 1 bis 3 Doppelbindungen und X für ein Alkalimetall oder Ammonium steht, enthalten.

7. Mikroemulsionskonzentrate nach Anspruch 1, **dadurch gekennzeichnet, dass** sie zusätzlich a6) Polyole, vorzugsweise Glycerin und/oder Konservierungsmittel und/oder Parfüm und/oder Salze und/oder pH-Regulantien enthalten.

8. Mikroemulsionskonzentrate nach Anspruch 1, **dadurch gekennzeichnet, dass** der Gesamtwassergehalt a5) 20 bis 25 Gew.-% - bezogen auf Konzentrat - beträgt.

9. Mikroemulsionskonzentrate nach einem oder mehreren der Ansprüche 1 bis 8, **dadurch gekennzeichnet, dass** sie enthalten
a1) 5 bis 20 Gew.-% Alkyl- und/oder Alkenylglykoside
a2) 3 bis 20 Gew.-% Co-Tenside ausgewählt aus der von Monoester des Glycerins mit einer C₆-C₂₂-Fettsäure gebildeten Gruppe
a3) 15 bis 35 Gew.-% Öle verschieden von a2)
a4) 4,75 bis 6,5 Gew.-% amphotere bzw. zwitterionische Tenside ausgewählt aus der Gruppe der Amphoacetate
a5) 15 bis 35 Gew.-% Wasser sowie
a6) 0,5 bis 50 Gew.-% Polyole, vorzugsweise Glycerin, und/oder Konservierungsmittel und/oder Parfüm und/oder Salze und/oder pH-Regulantien,
jeweils bezogen auf Konzentrat und in der Summe 100 Gew.-%.

10. Mikroemulsionskonzentrate nach Anspruch 9, **dadurch gekennzeichnet, dass** sie enthalten
a1) 7 bis 12 Gew.-% Alkyl- und/oder Alkenylglykoside
a2) 3 bis 7 Gew.-% Co-Tenside ausgewählt aus der von Monoester des Glycerins mit einer C₆-C₂₂-Fettsäure gebildeten Gruppe
a3) 15 bis 25 Gew.-% Öle verschieden von a2)
a4) 4,75 bis 6,5 Gew.-% amphotere bzw. zwitterionische Tenside ausgewählt aus der Gruppe der Amphoacetate
a5) 20 bis 30 Gew.-% Wasser sowie
a6) 25 bis 40 Gew.-% Polyole, vorzugsweise Glycerin, und/oder Konservierungsmittel und/oder Parfüm und/oder Salze und/oder pH-Regulantien,
jeweils bezogen auf Konzentrat ist und in der Summe 100 Gew.-%.

11. Verwendung einer Mischung aus a1) Alkyl- und/oder Alkenylglykosiden und a4) amphoteren und/oder zwitterionischen Tensiden ausgewählt aus der Gruppe der Amphoacetate als Emulgator- bzw. Tensidmischung zur Herstellung stabiler Mikroemulsionskonzentrate nach Anspruch 1 mit einem Gesamtwassergehalt von 0,5 bis 35 Gew.-%.

12. Verfahren zur Herstellung von Mikroemulsionskonzentraten nach Anspruch 1, **dadurch gekennzeichnet, dass** man eine Mischung herstellt aus a1) Alkyl- und/oder Alkenylglykosiden, a4) amphoteren oder zwitterionischen Tensiden ausgewählt aus der Gruppe der Amphoacetate in Anwesenheit von a5) Wasser und ggf. a6) Polyolen und/oder Konservierungsmittel und/oder Parfum und/oder Salze und/oder pH-Regulantien, diese erwärmt und dazu gleichzeitig oder nacheinander die a3) Öle verschieden von a2) sowie das Co-Tensid a2) ausgewählt aus der von Monoester des Glycerins mit einer C₆-C₂₂-Fettsäure gebildeten Gruppe zugibt.

13. Verwendung der Mikroemulsionskonzentrate nach einem der Ansprüche 1 bis 10 in Form seiner wässrigen Verdünnung als Imprägniermittel von Papier und Textil zur Herstellung von Flächengebilden in der Kosmetik, insbesondere sog. Wet Wipes.

14. Verwendung nach Anspruch 13, **dadurch gekennzeichnet, dass** es in Mengen von 0,1 bis 10 Gew.-% - bezogen auf wässrige Verdünnung - verwendet wird Konzentrat.

15. Imprägniermittel von Papier und Textil zur Herstellung von Flächengebilden in der Kosmetik enthaltend
A) 0,1 bis 10 Gew.-% Mikroemulsionskonzentrate nach Anspruch 1 und
B) Wasser sowie ggf.
C) weitere kosmetische Inhaltsstoffe ausgewählt aus der Gruppe der anionischen/nicht-ionischen, kationischen und/oder zwitterionischen Tenside sowie ggf.
D) weitere Zusatzstoffe verschieden von C).

## Claims

1. Microemulsion concentrates for impregnating textile and paper in cosmetics, comprising
a1) alkyl and/or alkenyl glycosides
a2) cosurfactants selected from the group formed of monoesters of glycerol with a C₆-C₂₂ fatty acid in quantities of 3 to 20 wt% - based on concentrate
a3) oils other than a2)
a4) amphoteric or zwitterionic surfactant selected from the group of amphoacetates in quantities of 4.75 to 6.5 wt% - based on concentrate - and
a5) water,
wherein the total water content is 0.5 - 35 wt% - based on concentrate.

2. The microemulsion concentrates according to claim 1, wherein they comprise
a1) alkyl and/or alkenyl glycosides of formula (I)
R¹O-[G]ₚ (I)
in which R¹ represents an alkyl and/or alkenyl radical having 4 to 22 carbon atoms,
G represents a sugar radical having 5 or 6 carbon atoms and p represents numbers from 1 to 10.

3. The microemulsion concentrates according to claim 1, wherein they comprise a2) monoesters of glycerol, selected from the group formed of monoesters having a C₆-C₂₂ fatty acid mixture comprising unsaturated C₁₂-C₂₂ fatty acids.

4. The microemulsion concentrates according to claim 1, wherein they comprise a3) oils that are formed from the group of esters of branched C₆-C₃₂ carboxylic acids with linear C₆-C₂₂ fatty alcohols, linear C₆-C₁₂ dialkyl ethers, symmetrical linear C₆-C₂₂ fatty alcohol dicarbonates, symmetrical branched C₆-C₂₂ dialkyl carbonates and/or saturated and linear "CX" hydrocarbons selected from the group comprising C7, C9, C11, C13, C15, C17, C19, C21 and/or C23 hydrocarbons.

5. The microemulsion concentrates according to claim 1, wherein they comprise a3) oils that are selected from the group formed of esters of C₆-C₂₂ fatty acids with branched C₆-C₃₂ carboxylic acids, linear C₆-C₁₂ dialkyl ethers and/or linear symmetrical C₆-C₂₂ fatty alcohol dicarbonates or mixtures thereof.

6. The microemulsion concentrates according to claim 1, wherein they comprise, as a4) amphoacetates, those of formula (II), 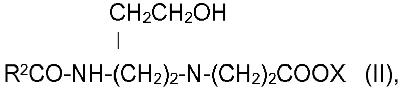 in which R2CO represents an aliphatic acyl radical having 6 to 22 carbon atoms and 0 or 1 to 3 double bonds, and X represents an alkali metal or ammonium.

7. The microemulsion concentrates according to claim 1, wherein they additionally comprise a6) polyols, preferably glycerol and/or preservatives and/or perfume and/or salts and/or pH regulators.

8. The microemulsion concentrates according to claim 1, wherein the total water content a5) is 20 to 25 wt% - based on concentrate.

9. The microemulsion concentrates according to one or more of claims 1 to 8, wherein they comprise
a1) 5 to 20 wt% of alkyl and/or alkenyl glycosides
a2) 3 to 20 wt% of cosurfactants selected from the group formed of monoesters of glycerol with a C₆-C₂₂ fatty acid
a3) 15 to 35 wt% of oils different to a2)
a4) 4.75 to 6.5 wt% of amphoteric and/or zwitterionic surfactants selected from the group of amphoacetates
a5) 15 to 35 wt% of water, and also
a6) 0.5 to 50 wt% of polyols, preferably glycerol, and/or preservatives and/or perfume and/or salts and/or pH regulators,
in each case based on concentrate and adding up to 100 wt%.

10. The microemulsion concentrates according to claim 9, wherein they comprise
a1) 7 to 12 wt% of alkyl and/or alkenyl glycosides
a2) 3 to 7 wt% of cosurfactants selected from the group formed of monoesters of glycerol with a C₆-C₂₂ fatty acid
a3) 15 to 25 wt% of oils different to a2)
a4) 4.75 to 6.5 wt% of amphoteric and/or zwitterionic surfactants selected from the group of amphoacetates
a5) 20 to 30 wt% of water, and also
a6) 25 to 40 wt% of polyols, preferably glycerol, and/or preservatives and/or perfume and/or salts and/or pH regulators,
is in each case based on concentrate and adding up to 100 wt%.

11. The use of a mixture of a1) alkyl and/or alkenyl glycosides and a4) amphoteric and/or zwitterionic surfactants selected from the group of amphoacetates as emulsifier or surfactant mixture for preparing stable microemulsion concentrates according to claim 1 having a total water content of 0.5 to 35 wt%.

12. A method for preparing microemulsion concentrates according to claim 1, wherein a mixture is prepared of a1) alkyl and/or alkenyl glycosides, a4) amphoteric or zwitterionic surfactants selected from the group of amphoacetates in the presence of a5) water and optionally a6) polyols and/or preservatives and/or perfume and/or salts and/or pH regulators, this is heated and, simultaneously or in succession, the a3) oils different to a2) and the cosurfactant a2) selected from the group formed of monoesters of glycerol with a C₆-C₂₂ fatty acid are added thereto.

13. The use of the microemulsion concentrates according to one of claims 1 to 10, in the form of the aqueous dilution thereof, as agent for impregnating paper and textile for the preparation of sheet-like articles in cosmetics, especially what are referred to as wet wipes.

14. The use according to claim 13, wherein it is used concentrate in quantities of 0.1 to 10 wt% - based on aqueous dilution.

15. An agent for impregnating paper and textile for the preparation of sheet-like articles in cosmetics, comprising
A) 0.1 to 10 wt% of microemulsion concentrates according to claim 1, and
B) water, and also optionally
C) further cosmetic ingredients selected from the group of anionic/nonionic, cationic and/or zwitterionic surfactants, and also optionally
D) further ingredients different to C).

## Revendications

1. Concentrés de microémulsions pour l'imprégnation de textile et papier dans la cosmétique, contenant
a1) des alkyl- et/ou alcénylglycosides
a2) des co-tensioactifs choisis dans le groupe constitué par les monoesters du glycérol avec un acide gras en C₆-C₂₂, en quantités de 3 à 20 % en poids - par rapport au concentré -
a3) des huiles différentes de a2)
a4) un tensioactif amphotère ou zwitterionique choisi dans le groupe des amphoacétates en quantités de 4,75 à 6,5 % en poids - par rapport au concentré - et
a5) de l'eau,
la teneur totale en eau valant de 0,5 à 35 % en poids - par rapport au concentré.

2. Concentrés de microémulsions selon la revendication 1, **caractérisés en ce qu'**ils contiennent a1) des alkyl- et/ou alcénylglycosides de formule (I)
R¹O-[G]ₚ (I)
dans laquelle R¹ représente un radical alkyle et/ou un radical alcényle, ayant de 4 à 22 atomes de carbone,
G représente un reste glucidique ayant 5 ou 6 atomes de carbone et p représente des nombres de 1 à 10.

3. Concentrés de microémulsions selon la revendication 1, **caractérisés en ce qu'**ils contiennent a2) des monoesters du glycérol, choisis dans le groupe qui est constitué par les monoesters avec un mélange d'acides gras en C₆-C₂₂ contenant des acides gras en C₁₂-C₂₂ insaturés.

4. Concentrés de microémulsions selon la revendication 1, **caractérisés en ce qu'**ils sont formés a3) des huiles choisies dans le groupe des esters d'acides carboxyliques en C₆-C₃₂ ramifiés avec des alcools gras linéaires en C₆-C₂₂, des éthers de dialkyle en C₆-C₁₂ linéaires, des dicarbonates d'alcools gras en C₆-C₂₂ linéaires symétriques, des carbonates de dialkyle en C₆-C₂₂ ramifiés symétriques et/ou des hydrocarbures « CX » linéaires et saturés, choisis dans le groupe comprenant les hydrocarbures en C7, C9, C11, C13, C15, C17, C19, C21 et/ou C23.

5. Concentrés de microémulsions selon la revendication 1, **caractérisés en ce qu'**ils contiennent a3) des huiles choisies dans le groupe constitué par les esters d'acides gras en C₆-C₂₂ avec des acides carboxyliques en C₆-C₃₂ ramifiés, les éthers de dialkyle en C₆-C₁₂ linéaires et/ou les dicarbonates d'alcools gras en C₆-C₂₂ symétriques linéaires ou des mélanges de ceux-ci.

6. Concentrés de microémulsions selon la revendication 1, **caractérisés en ce qu'**ils contiennent en tant que a4) amphoacétates ceux de formule (II), 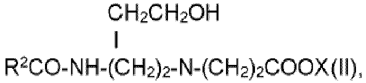 dans laquelle R2CO représente un radical acyle aliphatique ayant de 6 à 22 atomes de carbone et comportant 0 ou 1 à 3 doubles liaisons et X représente un métal alcalin ou l'ammonium.

7. Concentrés de microémulsions selon la revendication 1, **caractérisés en ce qu'**ils contiennent en outre a6) des polyols, de préférence du glycérol et/ou des conservateurs et/ou un parfum et/ou des sels et/ou des régulateurs du pH.

8. Concentrés de microémulsions selon la revendication 1, **caractérisés en ce que** la teneur totale en eau a5) vaut de 20 à 25 % en poids - par rapport au concentré.

9. Concentrés de microémulsions selon une ou plusieurs des revendications 1 à 8, **caractérisés en ce qu'**ils contiennent
a1) 5 à 20 % en poids d'alkyl- et/ou alcénylglycosides
a2) 3 à 20 % en poids de co-tensioactifs choisis dans le groupe constitué par les monoesters du glycérol avec un acide gras en C₆-C₂₂
a3) 15 à 35 % en poids d'huiles différentes de a2)
a4) 4,75 à 6,5 % en poids de tensioactifs amphotères ou zwitterioniques choisis dans le groupe des amphoacétates
a5) 15 à 35 % en poids d'eau ainsi que
a6) 0,5 à 50 % en poids de polyols, de préférence glycérol, et/ou conservateurs et/ou parfum et/ou sels et/ou régulateurs du pH,
chaque fois par rapport au concentré et en le total de 100 % en poids.

10. Concentrés de microémulsions selon la revendication 9, **caractérisés en ce qu'**ils contiennent
a1) 7 à 12 % en poids d'alkyl- et/ou alcénylglycosides
a2) 3 à 7 % en poids de co-tensioactifs choisis dans le groupe constitué par les monoesters du glycérol avec un acide gras en C₆-C₂₂
a3) 15 à 25 % en poids d'huiles différentes de a2)
a4) 4,75 à 6,5 % en poids de tensioactifs amphotères ou zwitterioniques choisis dans le groupe des amphoacétates
a5) 20 à 30 % en poids d'eau ainsi que
a6) 25 à 40 % en poids de polyols, de préférence glycérol, et/ou conservateurs et/ou parfum et/ou sels et/ou régulateurs du pH,
est chaque fois par rapport au concentré et en le total de 100 % en poids.

11. Utilisation d'un mélange de a1) alkyl- et/ou alcénylglycosides et a4) tensioactifs amphotères et/ou zwitterioniques choisis dans la groupe des amphoacétates, en tant que mélange émulsifiant ou tensioactif pour la préparation de concentrés de microémulsions stables selon la revendication 1, ayant une teneur totale en eau de 0,5 à 35 % en poids.

12. Procédé pour la préparation de concentrés de microémulsions selon la revendication 1, **caractérisé en ce qu'**on prépare un mélange à partir de a1) alkyl-et/ou alcénylglycosides, a4) tensioactifs amphotères et/ou zwitterioniques choisis dans la groupe des amphoacétates, en présence de a5) eau et éventuellement a6) polyols et/ou conservateurs et/ou parfum et/ou sels et/ou régulateurs du pH, on le chauffe et on y ajoute simultanément ou successivement les a3) huiles différentes de a2) ainsi que le co-tensioactif a2) choisi dans le groupe constitué par les monoesters du glycérol avec un acide gras en C₆-C₂₂.

13. Utilisation des concentrés de microémulsions selon l'une quelconque des revendications 1 à 10 sous forme de sa dilution aqueuse, en tant qu'agent d'imprégnation de papier et textile pour la fabrication de structures planes dans la cosmétique en, particulier de dénommés Wet Wipes.

14. Utilisation selon la revendication 13, **caractérisée en ce qu'**on utilisé en des quantités de 0,1 à 10 % en poids - par rapport à la dilution aqueuse le concentré.

15. Agent d'imprégnation de papier et textile destiné à la fabrication de structures planes dans la cosmétique, contenant
A) 0,1 à 10 % en poids de concentrés de microémulsions selon la revendication 1 et
B) de l'eau ainsi qu'éventuellement
C) d'autres composants cosmétiques choisis dans le groupe des tensioactifs anioniques/non ioniques, cationiques et/ou zwitterioniques ainsi qu'éventuellement
D) d'autres additifs différents de C).
